# EUROPÄISCHE PATENTSCHRIFT

(11) **EP 4 279 038 B1**
(45) Veröffentlichungstag und Bekanntmachung des Hinweises auf die Patenterteilung: **26.11.2025**
(21) Anmeldenummer: 23173882.4
(22) Anmeldetag: 17.05.2023
(51) Int. Cl.: A61F 2/966, A61B 1/00, A61B 1/018, A61B 1/313, A61F 2/04

(54) **STENT-ZUFÜHREINRICHTUNG UND ENDOSKOP MIT EINER SOLCHEN STENT-ZUFÜHREINRICHTUNG**
STENT DELIVERY DEVICE AND ENDOSCOPE WITH SUCH A STENT DELIVERY DEVICE
DISPOSITIF D'ALIMENTATION D'ENDOPROTHÈSE ET ENDOSCOPE ÉQUIPÉ D'UN TEL DISPOSITIF D'ALIMENTATION D'ENDOPROTHÈSE

(30) Priorität: 18.05.2022 DE 102022112498
(43) Veröffentlichungstag der Anmeldung: 22.11.2023
(73) Patentinhaber: Medi-Globe GmbH, 83101 Rohrdorf-Achenmühle (DE)
(72) Erfinder: EDEN, Ingo, 81479 München (DE)
(74) Vertreter: Hofstetter, Schurack & Partner

(56) Entgegenhaltungen:
- DE-A1- 102021 100 034
- US-A1- 2014 309 721
- US-A1- 2018 064 568

## Beschreibung

Die Erfindung betrifft eine Stent-Zuführeinrichtung und ein Endoskop mit einer solchen Stent-Zuführeinrichtung.

Derzeit in der Endoskopie gängige Stent-Zuführeinrichtungen bzw. Stent-Legesysteme bestehen aus einem Schlauch-in-Schlauch-System. Der innere Schlauch wird dabei als Führungskatheter bezeichnet, während der äußere Schlauch als Drückerkatheter (engl. "Pusher") bezeichnet wird. Der Führungskatheter weist dabei einen distalen Endbereich, an welchem der medizinische Stent anordenbar ist, einen proximalen Endbereich und einem Führungskanal auf, welcher sich zwischen dem distalen Endbereich und dem proximalen Endbereich erstreckt und wenigstens eine zwei Öffnungen zum Ein- und Ausführen eines Führungsdrahts besitzt. Der Drückerkatheter ist relativ zum Führungskatheter bewegbar, um den am Führungskatheter angeordneten Stent entlang des distalen Endbereichs des Führungskatheters vom Führungskatheter zu bewegen und den Stent freizugeben bzw. in einer Stenose abzulegen.

Der Führungsdraht wird dazu verwendet, den Führungskatheter und somit den geladenen Stent an seine gewünschte Position zu bringen. Dabei wird abhängig von der Art der Handhabung und der Länge des Führungsdrahtes prinzipiell zwischen Kurzdraht- und Langdrahtsystemen unterschieden. Der Hauptunterschied dabei ist der Umstand, dass bei einem Langdrahtsystem ein Katheterwechsel erfolgen kann, während das klinische Personal Zugriff auf den Führungsdraht hat. Im Gegensatz dazu wird beim Kurzdrahtsystem der Führungsdraht am Endoskop fixiert, so dass kein stetiges Nachführen des Führungsdrahtes durch das medizinische Personal erforderlich ist. Dadurch ist eine erhebliche Reduktion der Drahtlänge möglich, was zu verschiedenen Vorteilen führt, insbesondere im Hinblick auf eine verkürzte Dauer der gesamten Untersuchung und Behandlung sowie im Hinblick auf eine entsprechende Einsparung an Durchleuchtungszeit.

Bei Kurzdraht-geeigneten Stent-Zuführeinrichtungen wird der Führungsdraht beispielsweise in eine distale Spitze des Führungskatheters eingeführt und vor dem Stent durch eine Öffnung im Führungskatheter wieder ausgeführt. So besteht die Möglichkeit, den ausgeführten Führungsdraht am proximalen Ende des Schafts bzw. Arbeitskanals des Endoskops zu sichern. Dies hat den Nachteil, dass der Führungsdraht, um den Stent in einer Stenose abzulegen, zunächst entfernt werden muss, um den Stent ablegen zu können. Dies ist aber nicht nur ein zusätzlicher Arbeitsschritt, sondern es wird auch die sichere Führung für eventuell nachfolge Instrumente verloren.

Bei alternativen Stent-Zuführeinrichtungen wird der Führungsdraht distal in den Führungskatheter eingeführt und vor einem proximalen Ende des Führungskatheters wieder ausgeführt. Hierdurch ist es möglich, den Stent abzulegen, ohne den Führungsdraht zu entfernen. Diese Stent-Zuführeinrichtung besteht aber aus mehreren zusammengeschweißten Schläuchen und ist somit komplex und kostenintensiv in der Herstellung. Desweiten besteht beim Ablegen des Stents kein erkennbarer Widerstand im System, der erkennen lässt, wann der Stent tatsächlich abgelegt wurde. Hierdurch ist die Handhabung erschwert.

Die US 2018/064568 A1 offenbart Vorrichtungen zur Behandlung von gastrointestinalen Verengungen unter Verwendung von Kathetern zum schnellen Austausch von enteralen Stents. Der Katheter kann ein inneres Element und ein äußeres Element umfassen, wobei die beiden Elemente in Bezug aufeinander verschiebbar sind. In verschiedenen Ausführungsformen der Vorrichtung ist eine Rampe zum Herausführen eines Führungsdrahtes aus dem Katheter unter Verwendung von Teilen des äußeren Elements oder eines geformten Dorns vorgesehen. Das innere Element kann eine Reihe von Formen annehmen, einschließlich eines röhrenförmigen distalen Abschnitts, eines abgeschrägten oder integral angebrachten länglichen Mittelteils und eines proximalen Abschnitts. Ein Dorn kann in einem Abschnitt proximal der Führungsdrahtrampe verwendet werden, wobei der Dorn eine von mehreren offengelegten Formen annehmen kann.

Die DE 10 2021 100 034 A1 offenbart eine Stenteinsatzanordnung zur Verwendung mit einem Führungsdraht, die einen Gallenstent und einen gestreckten Stentförderschlauch umfasst, der einen Führungsdrahthalteabschnitt, der entsprechende distale und proximale Öffnungen, die einen Führungsdrahtweg durch diesen definieren, und einen längsgerichteten seitlich-passierbaren Teil umfasst. In einer Stentvorschiebe-Anordnung läuft der Führungsdraht durch die jeweiligen Öffnungen, um den Führungsdrahthalteabschnitt innen zu durchlaufen, und der Stent ist so angeordnet, dass er einen Stentförderschlauchabschnitt umgibt, der proximal vom Führungsdrahthalteabschnitt zum Vorschieben des Stents zusammen mit dem Stentförderschlauch entlang des Führungsdrahts in ein Körperlumen eines menschlichen Patienten versetzt ist. Wenn der Stent in der Stentvorschiebe-Anordnung an einem Zieleinsatzort innerhalb des Lumens angeordnet ist, bewirkt ein Zurückziehen des Stentförderschlauchs in proximaler Richtung, dass der Führungsdraht den seitlich-passierbaren Teil des Führungsdrahthalteabschnitts passiert, um den Führungsdraht vom Schlauch zu entkoppeln, ohne den Führungsdraht in Längsrichtung zu verschieben.

Aufgabe der vorliegenden Erfindung ist es, eine Stent-Zuführeinrichtung der eingangs genannten Art zu schaffen, welche eine verbesserte Ablegung eines medizinischen Stents ermöglicht. Eine weitere Aufgabe der Erfindung ist es, ein Endoskop mit einer derart verbesserten Stent-Zuführeinrichtung zu schaffen.

Die Aufgaben werden erfindungsgemäß durch eine Stent-Zuführeinrichtung mit den Merkmalen des Patentanspruchs 1 sowie durch ein Endoskop gemäß Patentanspruch 9 gelöst. Vorteilhafte Ausgestaltungen mit zweckmäßigen Weiterbildungen der Erfindung sind in den jeweiligen Unteransprüchen angegeben, wobei vorteilhafte Ausgestaltungen der Stent-Zuführeinrichtung als vorteilhafte Ausgestaltungen des Endoskops und umgekehrt anzusehen sind.

Ein erster Aspekt der Erfindung betrifft eine Stent-Zuführeinrichtung gemäß Anspruch 1, umfassend einen Führungskatheter mit einem distalen Endbereich, an welchem der zuzuführende Stent anordenbar ist, einem proximalen Endbereich und einem Führungskanal, welcher sich zwischen dem distalen Endbereich und dem proximalen Endbereich erstreckt und wenigstens eine erste Öffnung sowie eine zweite Öffnung für einen Führungsdraht besitzt. Weiterhin umfasst die Stent-Zuführeinrichtung einen Drückerkatheter (engl. "pusher"), welcher relativ zum Führungskatheter zwischen einer Ruhestellung, in welcher der Stent am distalen Endbereich des Führungskatheters haltbar ist, und einer Ablegestellung, in welcher der Stent freigebbar, bewegbar ist. Eine verbesserte Ablegung des Stents wird erfindungsgemäß dadurch erreicht, dass der Führungskatheter einen sich von der ersten Öffnung in Richtung der zweiten Öffnung des Führungskanals erstreckenden Schlitz aufweist und dass der Drückerkatheter eine Öffnung aufweist, durch welche der aus der ersten Öffnung des Führungskatheters geführte Führungsdraht aus dem Drückerkatheter führbar ist. Mit anderen Worten ist es erfindungsgemäß vorgesehen, dass der Führungskanal geschlitzt ist, wobei sich der Schlitz ausgehend von der ersten Öffnung in Richtung der zweiten Öffnung des Führungskanals, aber nicht notwendigerweise bis zur zweiten Öffnung erstreckt, sondern in den meisten Ausführungsformen in einem vorbestimmten Abstand zur zweiten Öffnung endet. Der Drückerkatheter ist an den Führungskatheter angepasst und umfasst seinerseits eine Öffnung, durch welche der aus der ersten Öffnung des Führungskatheters geführte Führungsdraht ebenfalls herausführbar ist, um zumindest abschnittsweise außerhalb des Führungskatheters und des Drückerkatheters zu verlaufen. Hierdurch kann der Führungsdraht beim relativen Bewegen bzw. Verschieben des Drückerkatheters gegenüber dem Führungskatheter zwischen der Ruhestellung und der Ablegestellung entlang des Schlitzes des Führungskatheters bewegt werden. Dies ermöglicht es, dass der Führungskatheter und der Drückerkatheter relativ zueinander verschoben werden können, obwohl der Führungsdraht aus beiden Kathetern geführt wurde. Damit ist auf konstruktiv einfache und kostengünstige Art ein Ablegen eines geladenen Stents bei liegendem Führungsdraht ermöglicht, ohne dass der Führungsdraht entfernt werden muss. Damit kann der Stent abgelegt werden, ohne die Führung für folgende Instrumente und dergleichen zu verlieren. So kann der Drückerkatheter in der Ablegestellung bleiben, das heißt in der Regel vor einer Stenose, während der Führungskatheter beispielsweise nach hinten bzw. zum Anwender (proximal, körperfern) gezogen wird. Hierbei kann der Führungsdraht durch den Schlitz von Öffnung zu Öffnung geführt werden. Während einer Operation befindet sich distal der Stent am distalen, körpernahen Ende des Drückerkatheters, während der Führungskatheter, auf dem der Stent angeordnet ist, nach proximal gezogen wird und so den Stent freigibt bzw. ablegt. Mit anderen Worten hält der Drückerkatheter beim relativen Zurückziehen des Führungskatheters den Stent am gewünschten Ort bis der Stent freigegeben und damit üblicherweise in einer Stenose abgelegt ist. Der Drückerkatheter überdeckt den Stent vorzugsweise zu keiner Zeit, das heißt weder in der Ruhestellung noch in der Ablagestellung noch in dazwischenliegen Stellungen. Der Drückerkatheter liegt in der Ruhestellung an einer proximalen Seite des Stents an oder in der Nähe der proximalen Seite des Stents und drückt beim Ablegen gegen diese Seite, um den Stent vom Führungskatheter zu schieben. Vorzugsweise fluchten die erste Öffnung des Führungskatheters und die Öffnung des Drückerkatheters zumindest bereichsweise bei in die Ruhestellung bewegtem Drückerkatheter, wodurch der Führungsdraht besonders einfach aus dem Führungs- und dem Drückerkatheter geführt werden kann. Der "distale" Bereich ist im Rahmen der vorliegenden Offenbarung jeweils derjenige Endbereich, der während der sachgerechten Anwendung in der Regel am Weitesten von dem medizinischen Fachpersonal entfernt ist. Mit anderen Worten bezeichnet "distal" denjenigen Bereich, der während der Operation näher am Patienten ist. Dies entspricht der fachüblichen Bezeichnung. Dementsprechend ist der "proximale" Bereich derjenige Bereich, der sich während der sachgerechten Anwendung in der Regel am Dichtesten am behandelnden Fachpersonal befindet. Anders ausgedrückt bezeichnet "proximal" denjenigen Bereich, der während der Operation weiter vom Patienten entfernt ist. Generell sind "ein/eine" im Rahmen dieser Offenbarung als unbestimmte Artikel zu lesen, also ohne ausdrücklich gegenteilige Angabe immer auch als "mindestens ein/mindestens eine". Umgekehrt können "ein/eine" auch als "nur ein/nur eine" verstanden werden.

In einer vorteilhaften Ausgestaltung der Erfindung ist vorgesehen, dass eine Länge des Schlitzes zwischen 130 mm und 180 mm, also beispielsweise 130 mm, 131 mm, 132 mm, 133 mm, 134 mm, 135 mm, 136 mm, 137 mm, 138 mm, 139 mm, 140 mm, 141 mm, 142 mm, 143 mm, 144 mm, 145 mm, 146 mm, 147 mm, 148 mm, 149 mm, 150 mm, 151 mm, 152 mm, 153 mm, 154 mm, 155 mm, 156 mm, 157 mm, 158 mm, 159 mm, 160 mm, 161 mm, 162 mm, 163 mm, 164 mm, 165 mm, 166 mm, 167 mm, 168 mm, 169 mm, 170 mm, 171 mm, 172 mm, 173 mm, 174 mm, 175 mm, 176 mm, 177 mm, 178 mm, 179 mm oder 180 mm beträgt. Vorzugsweise beträgt die Länge insbesondere 150 mm. Indem die Länge des Schlitzes zwischen 130 mm und 180 mm beträgt, können verschiedene gängige Stenttypen zuverlässig gehandhabt und abgelegt werden, wodurch die erfindungsgemäße Stent-Zuführeinrichtung für alle gängigen Operationsarten verwendet werden kann, die das Setzen von Stents beinhalten. Die Länge des Schlitzes entspricht dabei vorzugsweise einer überstehenden Länge des Führungskatheters gegenüber dem Drückerkatheter, wenn sich der Drückerkatheter in der Ruhestellung befindet.

In einer weiteren vorteilhaften Ausgestaltung der Erfindung ist es vorgesehen, dass eine Länge des Schlitzes einer maximalen Weglänge entspricht, welche der Drückerkatheter zum Freigeben des Stents relativ zum Führungskatheter zu bewegen ist. Hierdurch bildet das der ersten Öffnung des Führungskatheters gegenüberliegende Ende des Schlitzes eine Art Anschlag für den Führungsdraht, wodurch dem medizinischen Fachpersonal auf haptische Weise signalisiert wird, dass der maximale Verfahrweg bzw. die Ablegestellung erreicht ist, der Drückerkatheter nicht weiterbewegt werden kann und der Stent gelegt bzw. vollständig vom Führungskatheter abgeschoben wurde.

In einer weiteren vorteilhaften Ausgestaltung der Erfindung ist vorgesehen, dass der Führungsdraht und/oder der Führungskatheter wenigstens eine Markierung umfasst, welche eine vorbestimmte Weglänge der relativen Bewegung des Drückerkatheters gegenüber dem Führungskatheter signalisiert. Vorzugsweise wird die Markierung dabei für das medizinische Fachpersonal erst sichtbar, wenn die vorbestimmte Weglänge erreicht ist. Hierdurch kann dem medizinischen Fachpersonal eine optische Rückmeldung über den Verfahrweg des Drückerkatheters und damit über den Status des Ablegevorgangs gegeben werden.

In einer weiteren vorteilhaften Ausgestaltung der Erfindung ist vorgesehen, dass der Führungsdraht und/oder der Führungskatheter wenigstens eine Markierung aufweist, welche eine relative Weglänge signalisiert, die geringer ist als die maximale Weglänge und/oder einen unumkehrbaren Freigabepunkt des Stents signalisiert. Eine solche Markierung kann damit als Information bzw. Warnung für das behandelnde medizinische Fachpersonal dienen, dass bereits ein bestimmtes Teilstück der maximalen Weglänge für den Ablegevorgang erreicht wurde bzw. dass ein unumkehrbarer Freigabepunkt des Stents erreicht wurde, so dass dieser nur noch abgelegt, aber nicht mehr zurückgezogen werden kann.

Weiterhin kann es vorgesehen sein, dass der Führungsdraht und/oder der Führungskatheter wenigstens eine Markierung aufweist, welche die maximale Weglänge signalisiert. Hierdurch kann alternativ oder zusätzlich zu einer haptischen Rückmeldung über das Erreichen der Ablegestellung auch eine optische Rückmeldung an das behandelnde medizinische Fachpersonal gegeben werden.

In einer weiteren vorteilhaften Ausgestaltung der Erfindung ist ein Führungsrohr vorgesehen, welches im Bereich der Öffnung des Drückerkatheters anordenbar und/oder angeordnet ist und durch welches der aus dem Führungskatheter und dem Drückerkatheter geführte Führungsdraht durchführbar ist. Mit Hilfe eines derartigen Führungsrohrs, das auch durch die Öffnungen im Drückerkatheter und dem Führungskatheter bis in den Führungskanal eingebracht werden kann, ist ein einfaches Ausbringen des Führungsdrahts sichergestellt, da der Führungsdraht in das Führungsrohr gelegt werden kann und dann den Führungsdraht sicher aus dem Lumen des Führungskatheters führt.

Weitere Vorteile ergeben sich dadurch, dass das Führungsrohr aus Edelstahl, insbesondere aus chirurgischem Edelstahl, besteht. Hierdurch ist das Führungsrohr vor Korrosion durch Einwirkung von Blut und anderen Substanzen geschützt und besitzt eine kratzfeste Oberfläche, wodurch Kerben oder Markierungen, die Bakterien enthalten können, vermieden werden. Alternativ oder zusätzlich ist es vorgesehen, dass am Führungsrohr ein Benutzungshinweis, insbesondere als Fahne und/oder als Etikett, angeordnet ist. Mit Hilfe eines solchen Benutzungshinweises, beispielsweise einer am Edelstahlrohr befestigten, mit Informationen bedruckten Fahne, können einem Anwender Warn- und/oder Benutzungshinweise vermittelt werden. Ebenso kann ein solcher Benutzungshinweis den Ort des Austritts des Führungsdrahtes aus den Kathetern deutlich kennzeichnen.

In einer weiteren vorteilhaften Ausgestaltung der Erfindung ist vorgesehen, dass die Stent-Zuführeinrichtung eine Sicherungseinrichtung umfasst, mittels welcher der Führungsdraht reversibel an der Stent-Zuführeinrichtung festlegbar ist. Mit anderen Worten weist die Stent-Zuführeinrichtung eine Sicherungseinrichtung (engl. "Locking Device") auf, mit deren Hilfe der Führungsdraht - vorzugsweise an einem Endbereich - bedarfsweise an der Stent-Zuführeinrichtung lagegesichert und bedarfsweise wieder freigegeben werden kann. Dies stellt eine konstruktiv einfache Möglichkeit dar, um einerseits die korrekte Positionierung des Führungsdrahts sicherzustellen und um andererseits den Führungsdraht wieder entfernen zu können.

Ein zweiter Aspekt der Erfindung betrifft ein Endoskop, welches einen flexiblen Schaft umfasst, in welchem eine Stent-Zuführeinrichtung gemäß dem ersten Erfindungsaspekt angeordnet ist. Hierdurch ist eine verbesserte Ablegung eines medizinischen Stents ermöglicht. Weitere Merkmale und deren Vorteile sind den Beschreibungen des ersten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

In einer vorteilhaften Ausgestaltung ist vorgesehen, dass das Endoskop wenigstens eine Sicherungseinrichtung, insbesondere an einem Albarranhebel, umfasst, mittels welcher der Führungsdraht reversibel an einem Arbeitskanaleingang des Endoskops festlegbar ist. Mit anderen Worten weist das Endoskop eine Sicherungseinrichtung (engl. "Locking Device") auf, mit deren Hilfe der Führungsdraht im Bereich eines Arbeitskanaleingangs bedarfsweise lagegesichert und wieder freigegeben werden kann. Dies stellt eine konstruktiv einfache Möglichkeit dar, um einerseits die korrekte Positionierung des Führungsdrahts sicherzustellen und um andererseits den Führungsdraht wieder freigeben und entfernen zu können. Unter einem Albarranhebel wird ein Hebel an der distalen Spitze des Endoskops verstanden, der zur Steuerung der Stent-Zuführeinrichtung bzw. von anderen Kathetern, Koagulationssonden und dergleichen verwendet werden kann. Ein Albarranhebel wird hauptsächlich angewendet, wenn das Endoskop für die Urologie und/oder Gastroenterologie vorgesehen bzw. ausgebildet ist.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Beschreibung zu verlassen. Es sind somit auch Ausführungen als umfasst und offenbart anzusehen, die in den Figuren nicht explizit gezeigt und erläutert sind, jedoch durch separierte Merkmalskombinationen aus den erläuterten Ausführungen hervorgehen und erzeugbar

### Dabei zeigt:

Fig. 1 eine schematische Aufsicht einer erfindungsgemäßen Stent-Zuführeinrichtung;
Fig. 2 eine schematische Seitenansicht der erfindungsgemäßen Stent-Zuführeinrichtung;
Fig. 3 eine schematische Aufsicht der Stent-Zuführeinrichtung, wobei ein Führungskatheter bereichsweise aus einem Drückerkatheter gezogen ist;
Fig. 4 eine perspektivische Detailansicht des Führungskatheters und des Drückerkatheters, wobei sich der Drückerkatheter in einer Ruhestellung befindet und ein Führungsdraht aus den Führungs- und Drückerkathetern geführt ist;
Fig. 5 eine perspektivische Detailansicht des Führungskatheters und des teilgeschnittenen Drückerkatheters, wobei ein Schlitz des Führungskatheters erkennbar ist; und
Fig. 6 eine perspektivische Detailansicht einer weiteren Ausführungsform des Führungskatheters.

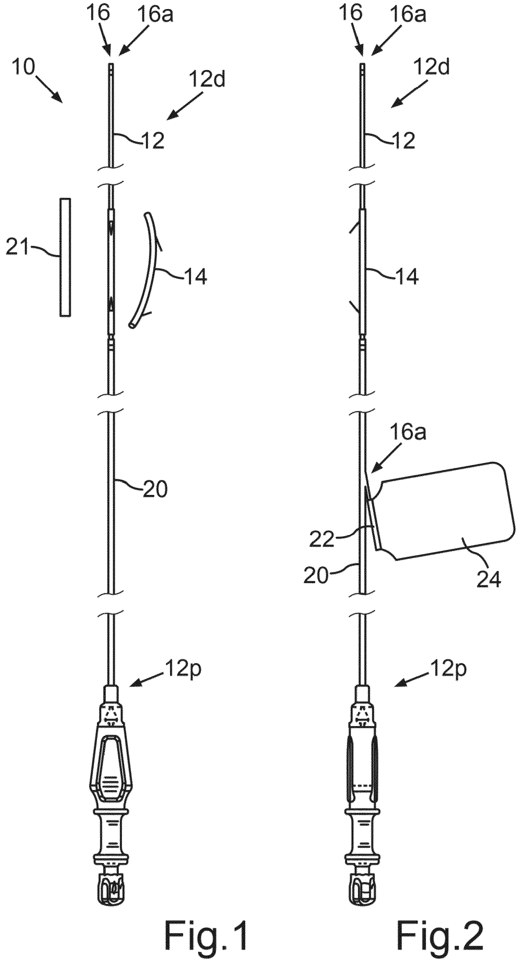

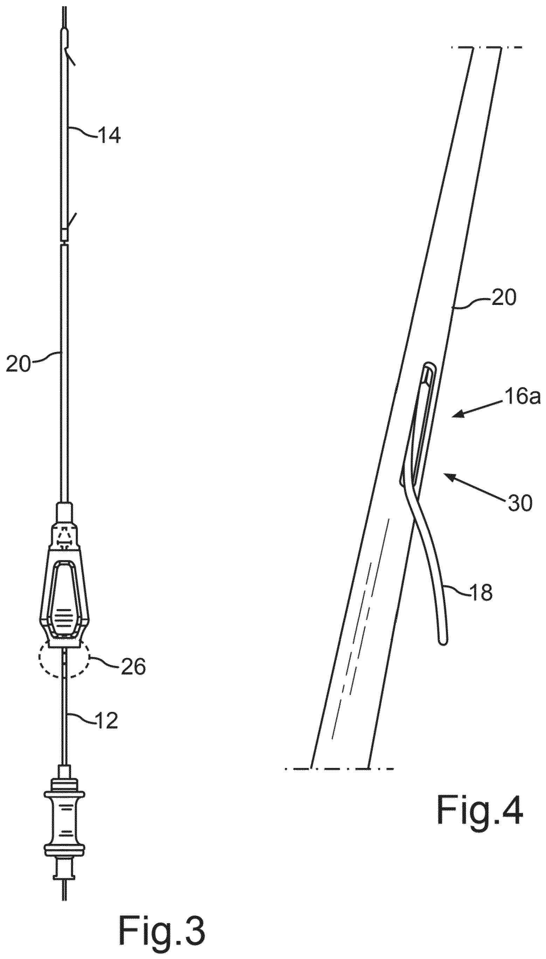

Fig. 1 zeigt eine schematische und verkürzte Aufsicht einer erfindungsgemäßen Stent-Zuführeinrichtung 10. Die Stent-Zuführeinrichtung 10 umfasst einen Führungskatheter 12 mit einem distalen Endbereich 12d, an welchem ein Stent 14 anordenbar ist, und mit einem proximalen Endbereich 12p. Der Führungskatheter 12 weist einen Führungskanal 16 auf, welcher sich zwischen dem distalen Endbereich 12d und dem proximalen Endbereich 12p erstreckt und für den Führungsdraht 18 wenigstens eine erste, proximale Öffnung 16a (s. Fig. 4) sowie eine zweite, distale Öffnung 16b besitzt. Die Stent-Zuführeinrichtung 10 umfasst weiterhin einen Drückerkatheter (pusher) 20, welcher relativ zum Führungskatheter 12 zwischen einer in Fig. 1 gezeigten Ruhestellung, in welcher der Stent 14 am distalen Endbereich 12d des Führungskatheters 12 gehalten ist, und einer Ablegestellung, in welcher der Stent 14 freigegeben bzw. vom Führungskatheter 12 geschoben und beispielsweise in einer Stenose abgelegt ist, bewegbar ist. In Fig. 1 ist zudem eine grundsätzlich optionale Stenteinführhilfe 21 abgebildet.

Fig. 2 zeigt eine schematische und verkürzt dargestellte Seitenansicht der in Fig. 1 abgebildeten Stent-Zuführeinrichtung 10. Man erkennt ein vorliegend aus Edelstahl bestehendes Führungsrohr 22, welches einenends im Bereich einer Öffnung 30 des Drückerkatheters 20 durch den Drückerkatheter 20 und die erste Öffnung 16a des Führungskatheters 12 geschoben und damit einenends im Führungskanal 16 platziert ist. Durch das Führungsrohr 22 kann der Führungsdraht 18 aus dem Führungskatheter 12 und dem Drückerkatheter 20 durchgeführt und damit aus dem Führungskanal 16 ausgeleitet werden. Am Führungsrohr 22 ist im vorliegenden Beispiel ein Benutzungshinweis 24 in Form einer Fahne bzw. eines Etiketts angeordnet, um das Ausbringen des Führungsdrahts 18 einfach zu gestalten. Wird nun der Führungsdraht 18 eingefügt, wird er in das Führungsrohr 22 gelegt und führt den Führungsdraht 18 aus dem Lumen bzw. dem Führungskanal 16 des Führungskatheters 12. Die grundsätzlich optionale Fahne 24 dient sowohl zur Auslenkung des Führungsdrahts 18 als auch als Markierung für den Ausgang. Zusätzlich verfügt sie über Warn- und/oder Bedienhinweise für den Anwender. Man sieht in Fig. 2 weiterhin, dass der Stent 14 auf dem Führungskatheter 12 angeordnet ist. Eine proximale Seite des Stents 14 liegt in der Ruhestellung an einer distalen Seite des Drückerkatheters 20 an. Der Stent 14 wird vom Drückerkatheter 20 in der Ruhestellung nicht überdeckt.

Fig. 3 zeigt eine schematische und verkürzte Aufsicht der Stent-Zuführeinrichtung 10, wobei der Führungskatheter 12 bereichsweise aus dem Drückerkatheter 20 gezogen ist, um den Drückerkatheter 20 von der in Fig. 1 und 2 gezeigten Ruhestellung in eine Ablegestellung zu bewegen und den Stent 14 vom Führungskatheter 12 zu schieben und beispielsweise in einer Stenose (nicht gezeigt) abzulegen. Mit anderen Worten hält der Drückerkatheter 20 beim relativen Zurückziehen des Führungskatheters 12 in proximale Richtung den Stent 14, bis der Stent 14 freigegeben ist. Hierdurch kann der Stent 14 am gewünschten Ort (d. h. üblicherweise in einer Stenose) abgelegt werden. Der Drückerkatheter 20 selbst verbleibt beim Ablegen bezüglich des Patienten zumindest im Wesentlichen ortsfest und drückt mit seiner distalen Seite gegen die proximale Seite des Stents 14, um den Stent 14 in Position zu halten, während der Führungskatheter 12 zurückgezogen wird. Man erkennt, dass der Drückerkatheter 20 den Stent 14 auch während der Bewegung von der Ruhestellung in der Ablagestellung zu keiner Zeit überdeckt. Dabei ist in der in Fig. 3 gezeigten Zwischenstellung eine erste Markierung 26 auf dem Führungskatheter 12 erkennbar, welche als Warnung für das baldige Erreichen einer maximalen, für das Ablegen des Stents 14 erforderlichen Weglänge dient. Sobald die maximale Weglänge erreicht wird, der Drückerkatheter 20 sich in der Ablegestellung befindet und der Stent 14 somit abgelegt ist, wird eine vorliegend nicht dargestellte zweite Markierung auf dem Führungskatheter 12 für das medizinische Fachpersonal sichtbar. Die maximale Weglänge und damit die Ablegestellung können beispielsweise nach einer relativen Bewegung zwischen Drückerkatheter 20 und Führungskatheter 12 von 150 mm erreicht sein.

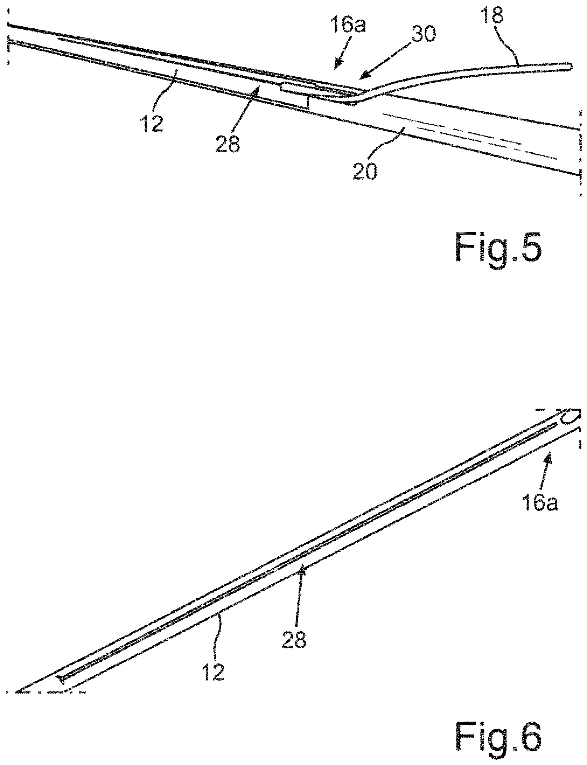

Fig. 4 zeigt eine perspektivische Detailansicht des Führungskatheters 12 und des Drückerkatheters 20, wobei sich der Drückerkatheter 20 in der Ruhestellung befindet und der Führungsdraht 18 aus dem Führungskatheter 12 und dem Drückerkatheter 20 geführt ist. Fig. 4 wird dabei in Zusammenschau mit Fig. 5 erläutert, wobei Fig. 5 eine perspektivische Detailansicht des Führungskatheters 12 und des teilgeschnittenen Drückerkatheters 20 im Bereich der ersten Öffnung 16a zeigt. Man erkennt insbesondere in Fig. 5, dass der Führungskatheter 12 einen sich von der ersten Öffnung 16a in Richtung der zweiten Öffnung 16b des Führungskanals 16 erstreckenden Schlitz 28 aufweist, welcher im gezeigten Ausführungsbeispiel eine Länge besitzt, die der maximalen Weglänge entspricht, welche der Drückerkatheter 20 relativ zum Führungskatheter 12 bewegbar sein soll, um den Drückerkatheter 20 aus der Ruhestellung in die Ablegestellung zu bringen. Im gezeigten Ausführungsbeispiel beträgt die Länge des Schlitzes daher ebenfalls 150 mm, wobei auch abweichende Längen vorgesehen sein können. Weiterhin ist die Öffnung 30 des Drückerkatheters 20 sichtbar, durch welche der aus der ersten Öffnung 16a des Führungskatheters 12 geführte Führungsdraht 18 aus dem Drückerkatheter 20 und gegebenenfalls in das optionale Führungsrohr 22 führbar ist. Dies ermöglicht es, dass der Führungskatheter 12 und der Drückerkatheter 20 relativ zueinander verschoben werden können, obwohl der Führungsdraht 18 aus beiden Schläuchen 12, 20 geführt wurde. In der in Fig. 4 gezeigten Ruhestellung des Drückerkatheters 20 fluchten die Öffnungen 16a, 30 zumindest im Wesentlichen, so dass der Führungsdraht 18 problemlos durch beide Öffnungen 16a, 30 geführt werden kann.

Beim relativen Bewegen des Drückerkatheters 20 gegenüber dem Führungskatheter 12 bzw. beim proximalen Herausziehen des Führungskatheters 12 aus dem Drückerkatheter 20 nimmt der Drückerkatheter 20 den Führungsdraht 18 mit und bewegt ihn entlang des Schlitzes 28 im Führungskatheter 12 in distaler Richtung bzw. in Richtung der distalen Öffnung 16a des Führungskanals, bis der Führungsdraht 18 am distalen Ende des Schlitzes 28 anstößt. Ist diese maximale Weglänge bzw. dieser maximale Verfahrweg erreicht, wird der Drückerkatheter 20 durch den Führungsdraht 18 blockiert und kann nicht weiter in diese Richtung bewegt werden. Dieser Widerstand ist für das medizinische Personal spürbar und signalisiert haptisch das Erreichen der Ablegestellung des Stents 14. Mit anderen Worten wird der gesicherte Führungsdraht 18 durch den Schlitz 28 geführt. Distal befindet sich der Stent 14 dabei am distalen Ende des Drückerkatheters 20, während der Führungskatheter 12, auf dem sich der Stent 14 in der Ruhestellung zunächst befindet, nach proximal gezogen wird und so den Stent 14 freigibt, sobald die Ablegestellung erreicht ist. So kann der Drückerkatheter 20 bei beispielsweise durch ein "Locking Device" gesichertem Führungsdraht 18 in Position bleiben (vor der Stenose), während der Führungskatheter 12 proximal zum Anwender gezogen wird, wie dies in Fig. 3 gezeigt ist.

Mit der erfindungsgemäßen Stent-Zuführeinrichtung 10 (engl. "Stent Positioning System") ist es daher möglich, bei liegendem und beispielsweise durch ein "Locking Device" gesichertem Führungsdraht 18 den Stent 14 abzulegen, ohne die Positionierung des Führungsdrahts 18 zu verlieren. Somit können, bei komplett entfernter Stent-Zuführeinrichtung 10 auch nachfolgende Instrumente über den weiterhin in Position liegenden Führungsdraht 18 korrekt in Position gebracht werden. Zudem wird das Erreichen der Ablegestellung haptisch und gegebenenfalls zusätzlich optisch durch eine oder mehrere Markierungen 26 signalisiert.

Fig. 6 zeigt eine perspektivische Detailansicht einer weiteren Ausführungsform des Führungskatheters 12. Der grundsätzliche Aufbau des Führungskatheters entspricht dabei dem vorherigen Ausführungsbeispiel. Im Unterschied zum vorherigen Ausführungsbeispiel ist der Schlitz 28 des Führungskatheters 12 im Bereich der ersten Öffnung 16a nicht aufgeweitet.

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, DIN-Toleranzen und dergleichen - als vom Rahmen der Erfindung mitumfasst anzusehen.

### Bezugszeichenliste:

- 10: Stent-Zuführeinrichtung
- 12: Führungskatheter
- 12d: distaler Endbereich
- 12p: proximaler Endbereich
- 14: Stent
- 16: Führungskanal
- 16a: erste Öffnung
- 16b: zweite Öffnung
- 18: Führungsdraht
- 20: Drückerkatheter
- 21: Stenteinführhilfe
- 22: Führungsrohr
- 24: Benutzungshinweis
- 26: Markierung
- 28: Schlitz
- 30: Öffnung

## Patentansprüche

1. Stent-Zuführeinrichtung (10), umfassend
- einen Führungskatheter (12) mit einem distalen Endbereich (12d), an welchem der Stent (14) anordenbar ist, einem proximalen Endbereich (12p) und einem Führungskanal (16), welcher sich zwischen dem distalen Endbereich (12d) und dem proximalen Endbereich (12p) erstreckt und wenigstens eine erste Öffnung (16a) sowie eine zweite Öffnung (16b) für einen Führungsdraht (18) besitzt, und
- einen Drückerkatheter (20), welcher relativ zum Führungskatheter (12) zwischen einer Ruhestellung, in welcher der Stent (14) am distalen Endbereich (12d) des Führungskatheters (12) haltbar ist, und einer Ablegestellung, in welcher der Stent (14) freigebbar, bewegbar ist,
**dadurch gekennzeichnet, dass**
- der Führungskatheter (12) einen sich von der ersten Öffnung (16a) in Richtung der zweiten Öffnung (16b) des Führungskanals (16) erstreckenden Schlitz (28) aufweist und
- der Drückerkatheter (20) eine Öffnung (30) aufweist, durch welche der aus der ersten Öffnung (16a) des Führungskatheters (12) geführte Führungsdraht (18) aus dem Drückerkatheter (20) führbar ist, wobei der Drückerkatheter (20) in der Ruhestellung an einer proximalen Seite des Stents (14) anliegt oder in der Nähe der proximalen Seite des Stents (14) liegt und beim Ablegen gegen die proximale Seite des Stents (14) drückt, um den Stent (14) vom Führungskatheter (12) zu schieben.

2. Stent-Zuführeinrichtung (10) nach Anspruch 1,
**dadurch gekennzeichnet, dass**
eine Länge des Schlitzes (28) zwischen 130 mm und 180 mm, insbesondere 150 mm, beträgt.

3. Stent-Zuführeinrichtung (10) nach Anspruch 1 oder 2,
**dadurch gekennzeichnet, dass**
eine Länge des Schlitzes (28) einer maximalen Weglänge entspricht, welche der Drückerkatheter (20) zum Freigeben des Stents (14) relativ zum Führungskatheter (12) zu bewegen ist.

4. Stent-Zuführeinrichtung (10) nach einem der Ansprüche 1 bis 3,
**dadurch gekennzeichnet, dass**
der Führungsdraht (18) und/oder der Führungskatheter (12) wenigstens eine Markierung (26) umfasst, welche eine vorbestimmte Weglänge der relativen Bewegung des Drückerkatheters (20) gegenüber dem Führungskatheter (12) signalisiert.

5. Stent-Zuführeinrichtung (10) nach Anspruch 4,
**dadurch gekennzeichnet, dass**
der Führungsdraht (18) und/oder der Führungskatheter (12) wenigstens eine Markierung (26) aufweist, welche eine relative Weglänge signalisiert, die geringer ist als die maximale Weglänge und/oder einen unumkehrbaren Freigabepunkt des Stents (14) signalisiert, und/oder dass der Führungsdraht (18) und/oder der Führungskatheter (12) wenigstens eine Markierung (26) aufweist, welche die maximale Weglänge signalisiert.

6. Stent-Zuführeinrichtung (10) nach einem der Ansprüche 1 bis 5,
**dadurch gekennzeichnet, dass**
ein Führungsrohr (22) vorgesehen ist, welches im Bereich der Öffnung (30) des Drückerkatheters (20) anordenbar und/oder angeordnet ist und durch welches der aus dem Führungskatheter (12) und dem Drückerkatheter (20) geführte Führungsdraht (18) durchführbar ist.

7. Stent-Zuführeinrichtung (10) nach Anspruch 6,
**dadurch gekennzeichnet, dass**
das Führungsrohr (22) aus Edelstahl besteht und/oder dass am Führungsrohr (22) ein Benutzungshinweis (24), insbesondere als Fahne und/oder als Etikett, angeordnet ist.

8. Stent-Zuführeinrichtung (10) nach einem der Ansprüche 1 bis 7,
**dadurch gekennzeichnet, dass**
diese eine Sicherungseinrichtung umfasst, mittels welcher der Führungsdraht (18) reversibel an der Stent-Zuführeinrichtung (10) festlegbar ist.

9. Endoskop, welches einen flexiblen Schaft umfasst, in welchem eine Stent-Zuführeinrichtung (10) nach einem der Ansprüche 1 bis 8 angeordnet ist.

10. Endoskop nach Anspruch 9,
**dadurch gekennzeichnet, dass**
dieses wenigstens eine Sicherungseinrichtung, insbesondere an einem Albarranhebel, umfasst, mittels welcher der Führungsdraht (18) reversibel an einem Arbeitskanaleingang des Endoskops festlegbar ist.

## Claims

1. A stent delivery device (10) comprising
- a guiding catheter (12) with a distal end portion (12d), at which the stent (14) can be arranged, a proximal end portion (12p) and a guiding channel (16), which extends between the distal end portion (12d) and the proximal end portion (12p) and comprises at least a first opening (16a) as well as a second opening (16b) for a guidewire (18), and
- a push catheter (20), which is movable in relation to the guiding catheter (12) between a rest position, in which the stent (14) can be retained at the distal end portion (12d) of the guiding catheter (12), and a deployment position, in which the stent (14) can be released,
**characterized in that**
- the guiding catheter (12) comprises a slit (28) extending from the first opening (16a) towards the second opening (16b) of the guiding channel (16), and
- the push catheter (20) comprises an opening (30), through which the guidewire (18) guided out of the first opening (16a) of the guiding catheter (12) can be guided out of the push catheter (20), wherein the push catheter (20) abuts on a proximal side of the stent (14) or lies in the vicinity of the proximal side of the stent (14) in the rest position, and presses against the proximal side of the stent (14) upon deployment to shift the stent (14) from the guiding catheter (12).

2. The stent delivery device (10) according to claim 1,
**characterized in that**
a length of the slit (28) is between 130 mm and 180 mm, in particular 150 mm.

3. The stent delivery device (10) according to claim 1 or 2,
**characterized in that**
a length of the slit (28) corresponds to a maximum travel length, by which the push catheter (20) is to be moved in relation to the guiding catheter (12) for releasing the stent (14).

4. The stent delivery device (10) according to any one of claims 1 to 3,
**characterized in that**
the guidewire (18) and/or the guiding catheter (12) include at least one marker (26), which indicates a predetermined travel length of the relative movement of the push catheter (20) with respect to the guiding catheter (12).

5. The stent delivery device (10) according to claim 4,
**characterized in that**
the guidewire (18) and/or the guiding catheter (12) comprise at least one marker (26), which indicates a relative travel length, which is less than the maximum travel length, and/or indicates an irreversible release point of the stent (14), and/or that the guidewire (18) and/or the guiding catheter (12) comprise at least one marker (26), which indicates the maximum travel length.

6. The stent delivery device (10) according to any one of claims 1 to 5,
**characterized in that**
a guiding tube (22) is provided, which can be arranged and/or is arranged in the area of the opening (30) of the push catheter (20) and through which the guidewire (18) guided out of the guiding catheter (12) and the push catheter (20) can be guided.

7. The stent delivery device (10) according to claim 6,
**characterized in that**
the guiding tube (22) is composed of stainless steel and/or that a usage instruction (24), in particular as a tag and/or as a label, is arranged at the guiding tube (22).

8. The stent delivery device (10) according to any one of claims 1 to 7,
**characterized in that**
it includes a securing device, by means of which the guidewire (18) can be reversibly fixed to the stent delivery device (10).

9. An endoscope, which includes a flexible shaft, in which a stent delivery device (10) according to any one of claims 1 to 8 is arranged.

10. The endoscope according to claim 9,
**characterized in that**
it includes at least one securing device, in particular at an Albarran lever, by means of which the guidewire (18) can be reversibly fixed to a working channel inlet of the endoscope.

## Revendications

1. Dispositif de pose d'endoprothèse (10), comprenant :
- un cathéter de guidage (12) ayant une zone d'extrémité distale (12d) sur laquelle l'endoprothèse (14) peut être disposée, une zone d'extrémité proximale (12p) et un canal de guidage (16) qui s'étend entre la zone d'extrémité distale (12d) et la zone d'extrémité proximale (12p) et possède au moins une première ouverture (16a) ainsi qu'une seconde ouverture (16b) pour un fil-guide (12), et
- un cathéter poussoir (20) qui est mobile par rapport au cathéter de guidage (12) entre une position de repos dans laquelle l'endoprothèse (14) peut être maintenue sur la zone d'extrémité distale (12d) du cathéter de guidage (12), et une position de dépose dans laquelle l'endoprothèse (14) peut être libérée,
**caractérisé en ce que**
- le cathéter de guidage (12) comporte une fente (28) s'étendant à partir de la première ouverture (16a) en direction de la seconde ouverture (16b) du canal de guidage (16) et
- le cathéter poussoir (20) comporte une ouverture (30) au moyen de laquelle le fil-guide (18) guidé à partir de la première ouverture (16a) du cathéter de guidage (12) peut être guidé à partir du cathéter poussoir (20), le cathéter poussoir (20) venant en butée, dans la position de repos, contre un côté proximal de l'endoprothèse (14) ou étant situé à proximité du côté proximal de l'endoprothèse (14) et pressant contre le côté proximal de l'endoprothèse (14) lors de la dépose afin de pousser l'endoprothèse (14) à partir du cathéter de guidage (12).

2. Dispositif de pose d'endoprothèse (10) selon la revendication 1,
**caractérisé en ce qu'**
une longueur de la fente (28) est comprise entre 130 mm et 180 mm, et est en particulier de 150 mm.

3. Dispositif de pose d'endoprothèse (10) selon la revendication 1 ou 2,
**caractérisé en ce qu'**
une longueur de la fente (28) correspond à une longueur de trajet maximale que le cathéter poussoir (20) doit parcourir par rapport au cathéter de guidage (12) afin de libérer l'endoprothèse (14).

4. Dispositif de pose d'endoprothèse (10) selon l'une des revendications 1 à 3,
**caractérisé en ce que**
le fil-guide (18) et/ou le cathéter de guidage (12) comprend au moins un marquage (26) indiquant une longueur de trajet prédéterminée du mouvement relatif du cathéter poussoir (20) par rapport au cathéter de guidage (12).

5. Dispositif de pose d'endoprothèse (10) selon la revendication 4,
**caractérisé en ce que**
le fil-guide (18) et/ou le cathéter de guidage (12) comporte au moins un marquage (26) qui indique une longueur de trajet relative inférieure à la longueur de trajet maximale et/ou un point de libération irréversible de l'endoprothèse (14), et/ou **en ce que** le fil-guide (18) et/ou le cathéter de guidage (12) comporte au moins un marquage (26) qui indique la longueur de trajet maximale.

6. Dispositif de pose d'endoprothèse (10) selon l'une des revendications 1 à 5,
**caractérisé en ce qu'**
un tube de guidage (22) est prévu, qui peut être disposé et/ou est disposé dans la zone de l'ouverture (30) du cathéter poussoir (20) et au moyen duquel le fil-guide (18) guidé à partir du cathéter de guidage (12) et du cathéter poussoir (20) peut être guidé.

7. Dispositif de pose d'endoprothèse (10) selon la revendication 6,
**caractérisé en ce que**
le tube de guidage (22) est en acier inoxydable et/ou **en ce qu'**une instruction d'utilisation (24), en particulier sous forme de languette et/ou d'étiquette, est disposée sur le tube de guidage (22).

8. Dispositif de pose d'endoprothèse (10) selon l'une des revendications 1 à 7,
**caractérisé en ce que**
celui-ci comprend un dispositif de blocage au moyen duquel le fil-guide (18) peut être fixé sur le dispositif de pose d'endoprothèse (10) de manière réversible.

9. Endoscope comprenant une tige souple dans laquelle est disposé un dispositif de pose d'endoprothèse (10) selon l'une des revendications 1 à 8.

10. Endoscope selon la revendication 9,
**caractérisé en ce**
**que** celui-ci comprend au moins un dispositif de blocage, en particulier sur un levier Albarran, au moyen duquel le fil-guide (18) peut être fixé de manière réversible sur une entrée de canal de travail de l'endoscope.
